# EUROPÄISCHE PATENTSCHRIFT

(11) **EP 2 765 216 B1**
(45) Veröffentlichungstag und Bekanntmachung des Hinweises auf die Patenterteilung: **01.11.2017**
(21) Anmeldenummer: 14154280.3
(22) Anmeldetag: 07.02.2014
(51) Int. Cl.: C23C 8/02, C23C 8/52, A61L 31/08

(54) **Verfahren zur Herstellung eines intravaskulären Funktionselements, Verwendung eines Salzbades zur Wärmebehandlung desselben**
Method for manufacturing an intravascular function element, use of a salt bath for its thermal treatment
Procédé de fabrication d' un élément fonctionnel intravasculaire, utilisation d'un bain de sels pour son traitement thermique

(30) Priorität: 11.02.2013 DE 102013101334
(43) Veröffentlichungstag der Anmeldung: 13.08.2014
(73) Patentinhaber: Acandis GmbH & Co. KG, 76327 Pfinztal (DE); ADMEDES GmbH, 75179 Pforzheim (DE)
(72) Erfinder: Schüßler, Andreas, 75179 Pforzheim (DE); Siekmeyer, Gerd, 76131 Karlsruhe (DE); Cattaneo, Giorgio, 76199 Karlsruhe (DE); Mailänder, Werner, 75331 Engelsbrand Grunbach (DE)
(74) Vertreter: Kilchert, Jochen

(56) Entgegenhaltungen:
- EP-A1- 1 522 605
- C. TREPANIER: "Effect of modification of oxide layer on NiTi stent corrosion resistance", JOURNAL OF BIOMEDICAL MATERIALS RESEARCH, Bd. 43, Nr. 4, 1. Dezember 1998 (1998-12-01), Seiten 433-440, XP055125080, ISSN: 0021-9304, DOI: 10.1002/(SICI)1097-4636(199824)43:4<433::A ID-JBM11>3.0.CO
- NEELAKANTAN L ET AL: "Selective surface oxidation and nitridation of NiTi shape memory alloys by reduction annealing", CORROSION SCIENCE, OXFORD, GB, Bd. 51, Nr. 3, 1. März 2009 (2009-03-01), Seiten 635-641, XP025962848, ISSN: 0010-938X, DOI: 10.1016/J.CORSCI.2008.12.018 [gefunden am 2009-02-23]
- Y. CHENG ET AL: "Surface characterization and mechanical property of TiN/Ti-coated NiTi alloy by PIIID", SURFACE AND COATINGS TECHNOLOGY, Bd. 201, Nr. 15, 27. August 2006 (2006-08-27), Seiten 6869-6873, XP055124943, ISSN: 0257-8972, DOI: 10.1016/j.surfcoat.2006.09.055

## Beschreibung

Die Erfindung betrifft ein Verfahren zur Herstellung eines Funktionselementes, insbesondere Stents sowie die Verwendung eines Salzbades zur Wärmebehandlung eines intravaskulären Funktionselements. Ferner wird ein intravaskuläres Funktionselement, insbesondere ein Stent offenbart.

In der Medizintechnik werden Stents in der Regel durch Laserverfahren hergestellt. Es kommen aber auch Geflechte aus Nitinol-Drähten für Implantate (z.B. Stents oder Okkluder) zum Einsatz. Anders als bei durch Laserverfahren hergestellten Stents, gleiten bei Drahtgeflechten die Drähte übereinander und ermöglichen daher eine gute Verformung der Stent-Strukturen. Grundsätzlich können (vaskuläre) Implantate aus Halbzeugen, wie Blechen, Präzisionsrohren oder Drähten gefertigt werden.

Beispielsweise beschreibt US 2004/0117001 A1 ein Verfahren zur Herstellung eines Stents aus Nitinol. Ein Ziel der US 2004/0117001 A1 besteht darin, den Nickel-Gehalt in einer oberflächennahen Schicht zu reduzieren, um zu verhindern, dass Nickel aus der Schicht freigesetzt wird, da dadurch die Biokompatibilität des Stents beeinträchtigt wird. Zur Herstellung der Stents wird ein Laser-Verfahren vorgeschlagen. Nach einem Kaltbearbeitungsschritt wird der Stent wärmebehandelt und dann bei Temperaturen unter 20°C elektropoliert. Zur thermischen Oxidierung wird der Stent mit Heißdampf bei einer Temperatur von 150°C für 12h beaufschlagt. Dadurch soll eine oxidische Oberfläche mit einem Ni-Gehalt von weniger als 2 Gew.% in einer Schichttiefe von 10 nm erreichbar sein.

Das bekannte Verfahren hat den Nachteil, dass die damit herstellbaren Oxidschicht bei Implantaten, die aus Drahtgeflechten bestehen, schnell verschleißen. Bei Geflechten kommt es darauf an, dass die Kontaktflächen der sich berührenden Drähte reibungsarm sind, gerade wenn man bedenkt, dass die implantierten Geflechte permanent pulsatilen Gefäßbewegungen ausgesetzt sind und sich die Drähte deshalb relativ zueinander bewegen. Ein niedriger Reibungskoeffizient der Drahtoberflächen ist überdies für die gute Bewegbarkeit des Implantats in einem Zufuhrkatheter wichtig. Weitere Verfahren zur Herstellung von intravaskulären Funktionselementen sind beispielhaft aus C. Trepanier: "Effect of modification of oxide layer on NiTi Stent Corrosion Resistance" (1998), L. Neelakantan: "Selective surface oxidation and nitridation of NiTi shape memory alloys by reduction annealing" (2009), Y. Cheng: "Surface characterization and mechanical property of TiN/Ti-coated NiTi alloy by PIIID" oder EP 1 522 605 A1 bekannt.

Es ist Aufgabe der Erfindung, ein Verfahren vorzuschlagen, mit dem ein intravaskuläres Funktionselement, insbesondere Stent oder intravaskuläres Coil oder intravaskuläres Verschlussdevice, mit verbessertem Verschleiß- und Reibungsverhalten und guter Biokompatibilität herstellbar ist. Eine weitere Aufgabe der Erfindung ist es, die Verwendung eines Salzbades zur Wärmebehandlung eines intravaskulären Funktionselements vorzuschlagen.

Diese Aufgabe wird im Hinblick auf das Verfahren durch den Gegenstand des Anspruches 1 und im Hinblick auf die Verwendung durch den Gegenstand des Anspruchs 11 gelöst.

Insbesondere wird die Aufgabe durch ein Verfahren zur Herstellung eines intravaskulären Funktionselementes gelöst, das in ein Hohlorgan einführbar ist und wenigstens einen Draht aus einer Legierung umfasst, die die Legierungselemente Nickel und Titan aufweist. Bei dem Verfahren wird ein Metallkörper des Drahtes mit metallischer Oberfläche bereitgestellt. Dann wird eine erste Oxidschicht auf der metallischen Oberfläche des Metallkörpers ausgebildet. Zur thermischen Ausbildung einer zweiten Mischoxidschicht auf der ersten Oxidschicht wird eine Wärmebehandlung des Drahtes in einem Stickstoff enthaltenden Salzbad durchgeführt, wobei die Schritte zum Ausbilden der ersten und zweiten Oxidschicht derart ausgeführt werden, dass die Gesamtschichtdicke von 15nm bis 100nm beträgt und die Mischoxidschicht TiO₂ und wenigstens Titanoxinitrid und/oder Titanitrid aufweist. Hierbei enthält das Salzbad ein Gemisch aus Kaliumnitrat und Natriumnitrit, wobei der Anteil an Kaliumnitrat größer als der Anteil an Natriumnitrit ist.

Für die Bereitstellung des Drahtmetallkörpers mit metallischer Oberfläche wird eine Vorbehandlung durchgeführt, bei der eine üblicherweise auf der Drahtoberfläche vorhandene Oxidschicht entfernt wird. Diese Oxidschicht mit einer Dicke von 0,2µm bis 5µm entsteht bei der Drahtherstellung, wenn zur Einstellung der Materialeigenschaften des Drahtes eine Wärmebehandlung durchgeführt wird. Für die Entfernung der Oxidschicht kommen verschiedene Verfahren in Frage, die im Rahmen der Erfindung eingesetzt werden können, um einen Drahtmetallkörper mit metallischer Oberfläche bereitszustellen. Die Erfindung ist nicht auf diese Verfahren beschränkt.

Bei einer bevorzugten Ausführung wird die obige herstellungsbedingte Oxidschicht durch Elektropolieren entfernt. Dabei wird in an sich bekannter Weise in einem Elektrolytbad unter Einwirkung von Strom von der Drahtoberfläche Metall zusammen mit Verunreinigungen sowie einer auf der Drahtoberfläche natürlich gebildeten Oxidschicht abgetragen, so dass nach dem Elektropolieren eine glatte und homogene metallische Oberfläche vorliegt, die im Wesentlichen oxidfrei ist.

Der Drahtmetallkörper mit metallischer Oberfläche kann auf andere Weise erzeugt werden, bspw. durch chemisches oder elektrochemisches oder mechanisches Abtragen der Oberflächenschicht des Drahtes. Eine Möglichkeit des chemischen Abtragens ist das Ätzen oder Beizen. Dabei wird die natürliche Oxidschicht entfernt, so dass die metallische Oberfläche des Drahtkörpers freiliegt. Die hierfür verwendeten Prozessparameter sind dem Fachmann bspw. aus US2004/0117001 A1 bekannt. Außerdem sind mikroabrasive Antragsverfahren bekannt, die ebenfalls bei der Erfindung eingesetzt werden können.

Auf der metallischen Oberfläche des Drahtmetallkörpers wird eine erste Oxidschicht ausgebildet, auf die die zweite Mischoxidschicht thermisch aufgebracht wird. Die Bildung der ersten Oxidschicht kann im einfachsten Fall in Form einer natürlichen Oxidschicht erfolgen, die entsteht, wenn die metallische Oberfläche des Drahtmetallkörpers der Umgebungsluft ausgesetzt wird. Wenn die Vorbehandlung des Drahtes bspw. durch Elektropolieren erfolgt, hat sich gezeigt, dass die natürliche, erste Oxidschicht eine Dicke von ca. 3nm bis 10nm aufweist.

Der Drahtquerschnitt ist nicht auf eine bestimmte Form beschränkt. Möglich sind runde, insbesondere kreisrunde, elliptische, eckige oder andere Querschnittsformen des Drahtes.

Die Erfindung hat mehrere Vorteile.

Die auf der Drahtoberfläche gebildete Oxidschicht ist nickelarm und enthält TiO₂, wodurch das Korrosionsverhalten und die Biokompatibilität des Implantats verbessert werden. Die Ausbildung der Oxidschicht als Mischoxidschicht, in der wenigstens Titanitrid und/oder Titanoxinitrid vorhanden ist, erhöht die Schichthärte, wodurch der Verschleiß bei Beanspruchung des Funktionselementes, insbesondere des Implantats, im Gefäß verringert wird. Dieser Vorteil kommt besonders bei Geflechten, wie bei geflochtenen Stents zum Tragen, bei denen Drähte sich berühren und aufeinander gleiten. Damit wird die Qualität des Funktionselementes, insbesondere des Implantats verbessert, bspw. mit Blick auf die Compliance im Gefäß. Überdies wird der Reibungskoeffizient der Oberläche des Funktionselementes, insbesondere des Implantats verringert, was zu einem verbesserten Gleitverhalten im Katheter führt. Die guten Gleiteigenschaften wirken einerseits zwischen den Drähten selbst, wodurch die Crimpbarkeit, d.h. die Fähigkeit des Funktionselementes komprimiert zu werden, verbessert wird. Andererseits wirken die guten Gleiteigenschaften zwischen den Drähten und der Katheterinnenwand. Die dadurch verringerte Schiebekraft, die zum Bewegen des Funktionselements, insbesondere Implantats im Katheter erforderlich ist, erhöht die Sicherheit, da das Risiko des Blockierens und der Beschädigung des Funktionselements, insbesondere Implantats im Katheter verringert wird. Gleiches gilt für Zuführsysteme, bei denen die Zuführung des Funktionselements nicht durch Bewegung des Funktionselements selbst sondern durch eine Relativbewegung zwischen einem Teil des Zuführsystems und dem Funktionselement bewirkt wird.

Die Schichtdicke von 10nm bis 100nm hat den Vorteil, dass die Abriebfestigkeit im Vergleich zu einem Draht, der nur elektropoliert ist, verbessert wird. Die natürliche Oxidschicht, die sich bspw. nach einer Elektropolitur einstellt, hat eine Schichtdicke von ca. 3-10nm, weshalb die Schicht leicht abgerieben werden kann.

Die erfindungsgemäß erhöhte Schichtdicke zusammen mit der Nitridierung der Oxidschicht verbessert die Abriebfestigkeit.

Im Vergleich zu herkömmlichen Oxidschichten auf NiTi-Drähten mit einer Dicke von mehr als 200nm, die sich bei der Drahtherstellung einstellen, ist die erfindungsgemäße Mischoxidschicht schützender gegen den Austritt von Nickel-Ionen, was sich bspw. an dem guten Korrosionsverhalten der Schicht zeigt. Die Erfindung vereint daher die guten Schutzeigenschaften einer sehr dünnen Oxidschicht mit der guten Abriebfestigkeit einer relativ dicken Schicht sowie mit guten Gleiteigenschaften.

Vorteilhafterweise beträgt die Gesamtschichtdicke, d.h. die Dicke der ersten und zweiten Schicht mindestens 30nm, insbesondere mindestens 35nm, insbesondere mindestens 40nm, insbesondere mindestens 45nm, insbesondere mindestens 50nm, insbesondere mindestens 55nm. Mit der Erhöhung der Untergrenze des Schichtdickenbereiches wird die Abriebfestigkeit weiter verbessert.

Wenn die Gesamtschichtdicke höchstens 95nm, insbesondere höchstens 90nm, insbesondere höchstens 85nm, insbesondere höchstens 80nm, insbesondere höchstens 75nm, insbesondere höchstens 70nm, insbesondere höchstens 65nm, insbesondere höchstens 60nm beträgt, werden durch die Verringerung der Obergrenze des Schichtdickenbereiches die schützenden Eigenschaften der Schicht verbessert. Außerdem sinkt das Risiko, dass sich - insbesondere bei Deformationen der Drähte - Schichtbestandteile von der Drahtoberfläche löst bzw. die Schicht brüchig wird.

Die vorstehend genannten Werte für die Untergrenze und die Obergrenze können jeweils miteinander kombiniert werden, um eine gezielte Verbesserung der jeweiligen Schichteigenschaften einzustellen. Bspw. kann die Untergrenze für die obigen Maximalwerte 30nm betragen. Ein besonders vorteilhafter Bereich der Schichtdicke beträgt von 20nm bis 90nm, insbesondere von 30nm bis 90nm, insbesondere von 40nm bis 80nm, insbesondere von 50nm bis 70nm.

Bei einer bevorzugten Ausführungsform bildet der Peak der Sauerstoffkonzentration in der Mischoxidschicht ein Plateau. Bspw. kann das Plateau in einer Schichttiefe von 5nm bis 50nm, insbesondere von 10nm bis 40nm, insbesondere von 15nm bis 30nm ausgebildet sein. Die Gesamtschichtdicke beträgt dabei mindestens ca. 60nm. Als Grund für das Plateau wird angenommen, dass der Sauerstoff sich nach Außen zur Schichtoberfläche hin bevorzugt mit Stickstoff und nach Innen zum Drahtmaterial hin mit Titan verbindet.

Das Verhältnis der Intensitäten zwischen Stickstoff und Sauerstoff (N/O) beträgt im Bereich des Sauerstoffplateaus höchstens 1:2,0, insbesondere von 1:2,5 bis 1:10, und nimmt zum Drahtmetallkörper hin ab, wobei die Intensität jeweils durch Augerelektronenspektroskopie (AES) im Tiefenprofile durch die Mischoxidschicht ermittelt wird. An der Schichtoberfläche beträgt das Verhältnis der Intensitäten zwischen Stickstoff und Sauerstoff (N/O) maximal ca. 1:2,0, insbesondere ca. 1:2,5. Das Verhältnis 1:2,0 liegt in der unmittelbaren Randschicht der Schichtaußenfläche (ca. 5nm-10nm), das Verhältnis 1:6 in etwa bei einer Schichttiefe von ca. 20-25nm und das Verhältnis 1:10 in etwa bei einer Schichttiefe von ca. 35-40 nm vor. Allgemein nimmt also das Verhältnis N/O zum Drahtmetallkörper hin ab.

Da Sauerstoff nicht im Drahtmaterial, sondern nur in der Schicht vorhanden ist, wird die Stickstoffintensität der Mischoxidschicht in Bezug auf die Sauerstoffintensität angegeben. Dadurch lässt sich indirekt der Stickstoffgehalt der Mischoxidschicht im Bereich der Schichtdicke charakterisieren.

AES wird bekanntermaßen zur Analyse der in einer oberflächennahen Schicht enthaltenen Elemente eines Materials eingesetzt. Durch sukzessiven Abtrag der Schicht durch Sputtern wird durch die AES-Analyse der jeweils freigelegten Schichtoberfläche ein Tiefenprofil der Elementverteilung in der Schicht erzeugt, das zur Charakterisierung des Stickstoffgehaltes bezogen auf den Sauerstoffgehalt sowie zum Nachweis des Konzentrationsverlaufs der übrigen Elemente, wie Ni und Ti herangezogen wird. Die gemessene Intensität des jeweiligen Elementes ergibt sich in bekannter Weise aus den bei der AES-Analyse durch Elektronenbeschuss emittierten Auger-Elektronen.

Vorzugsweise liegt der Stickstoff bis in eine Tiefe der Mischoxidschicht von bis zu 2/6, insbesondere bis zu 3/6, insbesondere bis zu 4/6 der Gesamtdicke der Mischoxidschicht einschließlich der ersten Oxidschicht vor. In absoluten Werten geht die stickstoffhaltige Randschicht bis in eine Schichttiefe von 10nm, insbesondere 20nm, insbesondere 30nm, insbesondere 40nm, insbesondere 50nm, bspw. bei einer Mischoxidschicht mit einer Gesamtdicke von ca. 60nm. Dadurch wird eine harte Randschicht der Mischoxidschicht erzeugt und das Abriebverhalten verbessert.

Vorzugsweise wird der Draht zur Herstellung eines Drahtgebildes vor der Wärmebehandlung verformt, wobei sich wenigstens ein Abschnitt, insbesondere mehrere Abschnitte des verformten Drahtes überkreuzen und/oder berühren. Das Drahtgebilde kann bspw. ein Geflecht, konkret ein geflochtenes Implantat, bspw. ein geflochtener Stent, ein Graft-Stent, ein geflochtenes Okklusionsdevice oder ein geflochtener Flow Diverter umfassen. Für das Geflecht werden in bekannter Weise Maschen gebildet, die durch Drahtüberkreuzungen begrenzt sind. Das Geflecht kann aus einem einzigen Draht oder aus mehreren Drähten gebildet sein. Dabei können herkömmliche Flechttechniken verwendet werden. Im Bereich der Drahtüberkreuzungen können sich die Drähte bzw. Abschnitte berühren oder voneinander beabstandet sein.

Das Drahtgebilde kann ein intravaskuläres Coil bspw. zur Aneurysmaverödung umfassen. Derartige Coils weisen einen oder mehrere spiralförmig gewickelte Drähte auf, die sich zumindest abschnittsweise berühren und Kontaktstellen bilden.

Die bevorzugte Ausbildung des Drahtgebildes nach dem Elektropolieren des Drahtes bzw. allgemein vor der Wärmebehandlung zur Bildung der Mischoxidschicht hat den Vorteil, dass der Draht in der für das Elektropolieren eingesetzten Lösung im geraden Zustand, d.h. ohne Drahtüberkreuzung oder Kontaktstelle, behandelt wird. Damit wird erreicht, dass die Lösung die Drahtoberfläche gleichmäßig im gesamten Oberflächenbereich benetzt. Eine Abschattung im Bereich von Drahtüberkreuzungen und/oder Kontaktstellen wird vermieden, da die Ausbildung des Drahtgebildes erst nach dem Elektropolieren erfolgt. Durch den dadurch erreichbaren gleichmäßigen Abtrag beim Elektropolieren wird die Voraussetzung für die Ausbildung einer homogenen und möglichst konstant dicken Oxidschicht in den nachfolgenden Prozessschritten geschaffen.

Konkret wird durch das Elektropolieren die nach der Drahtherstellung vorhandene Oxidschicht samt Verunreinigungen entfernt, so dass nach dem Elektropolieren zunächst eine im wesentlichen blanke Metalloberfläche des Drahtes vorliegt. Der Draht wird aus der Lösung entfernt und der Umgebungsluft ausgesetzt. Durch den Kontakt mit Luft bildet sich eine natürliche Oxidschicht mit einer Dicke von ca. 5nm an der Drahtoberfläche. Diese Oxidschicht ist homogen und weist eine im Wesentlichen konstante Dicke auf. Sie besteht hauptsächlich aus TiO₂. Der Ni-Gehalt in der Oxidschicht nimmt zur Oberfläche hin stark ab, die im Wesentlichen nickelfrei ist.

Nach dem Umformen des Drahtes zu einem Drahtgebilde mit wenigstens einer Überkreuzung erfolgt die thermische Oxidierung im Salzbad. Dadurch wird die Oberfläche nach dem Elektropolieren modifiziert, insbesondere passiviert und gehärtet. Da die nach dem Elektropolieren natürlich ausgebildete Oxidschicht zumindest im oberflächennahen Grenzbereich nickelarm oder sogar nickelfrei ist und somit wie eine Barriere zur Metallgrenzfläche des Drahtes wirkt, enthält die thermische ausgebildete Oxidschicht ebenfalls nur einen geringen Ni-Gehalt bzw. ist zumindest im oberflächennahen Grenzbereich nickelarm oder sogar nickelfrei. Durch die sich anschließende thermische Behandlung im stickstoffhaltigen Salzbad wird auf der natürlich ausgebildeten Oxidschicht eine dichte Mischoxidschicht erzeugt, die TiO₂ enthält. Zusätzlich enthält die Mischoxidschicht Anteile an Stickstoff, welcher als Titanoxinitrid und/oder Titanitrid gebunden ist. Titanoxinitrid und/oder Titanitrid ergibt sich aus dem Salzbad, bspw. bei einer Verwendung eines Alkalimetall-Stickstoff-Salzes, insbesondere Kaliumnitrat oder Natriumnitrit oder einem Gemisch aus Kaliumnitrat und Natriumnitrit, ergibt. Das thermisch ausgeformte Nitrid, insbesondere Titanoxinitrid und/oder Titaninitrid, wirkt als Hartstoff, der die Schichthärte erhöht und das Verschleiß- und Reibungsverhalten des Funktionselemetes, insbesondere Implantats verbessert.

Im Gegensatz zum Stand der Technik wird bei dieser Ausführung also zuerst eine Elektropolitur durchgeführt und anschließend eine Wärmebehandlung im Salzbad. Insgesamt wird durch die thermische (inerte) Salzbadbehandlung eine Oxidierung im Anschluss an den elektrochemischen Polierprozess realisiert. Dadurch sind sehr dichte, sowie reibungsarme und verschleißfeste Oxidschichten, die dicker als 10 nm sein können, herstellbar. Außerdem können besonders günstige physikalische, beispielsweise hinsichtlich der Radialkraft und des Ermüdungsverhaltens, physikalisch-chemische, insbesondere bezüglich der Nickel-Freisetzung und des Korrosionsverhaltens, und biologische Grenzflächencharakteristika, beispielsweise die Thrombogenität, von Funktionselementen, insbesondere Implantaten wie Stents, vergleichsweise genau und einfach eingestellt werden. Dadurch lässt sich das Oberflächenverhalten und die Biokompatibilität signifikant verbessern.

Die Erfindung ist nicht auf eine spezielle NiTi-Legierung eingeschränkt, sondern allgemein bei den in der Medizintechnik gebräuchlichen NiTi-Legierungen einsetzbar, aus denen intravaskuläre Funktionselemente, insbesondere Implantate hergestellt werden, deren Oberflächen durch eine Oxidschicht geschützt werden soll.

Als Legierung kommen bspw. verschiedene binäre Verbindungen auf Ni-Basis in Frage, wie bspw. NiTi Legierungen, insbesondere Nitinol (Ni 55 Gew.-%, Ti 45 Gew.-%), oder verschiedene ternäre Verbindungen wie bspw. NiTiFe oder NiTiNb oder NiTiCr oder quarternäre Legierungen wie NiTiCoCr.

Der Draht kann mindestens 5 Gew.-%, vorzugsweise mindestens 10 Gew.-%, vorzugsweise mindestens 20 Gew.-%, vorzugsweise mindestens 40 Gew.-%, Nickel aufweisen. Der Draht kann weiterhin vorzugsweise höchstens 80 Gew.-%, vorzugsweise höchstens 60 Gew.-%, vorzugsweise höchstens 55 Gew.-% vorzugsweise höchstens 50 Gew.-% Nickel aufweisen. Der Titangehalt kann vorzugsweise mindestens 10 Gew.-%, vorzugsweise mindestens 30 Gew.-%, vorzugsweise mindestens 40 Gew.-%, vorzugsweise mindestens 50 Gew.-%, betragen. Eine Obergrenze für den Titangehalt kann 90 Gew.-%, vorzugsweise 80 Gew.-%, vorzugsweise 65 Gew.-%, vorzugsweise 60 Gew.-%, vorzugsweise 55 Gew.-%, betragen.

Vorzugsweise erfolgt die Wärmebeaufschlagung (zumindest teilweise) gleichzeitig mit dem Eintauchen in das Salzbad, weiter vorzugsweise erfolgt zumindest während 10%, weiter vorzugsweise zumindest während 30%, noch weiter vorzugsweise zumindest 50%, noch weiter bevorzugt zumindest 90% der Salzbad-Eintauchzeit eine Wärmebeaufschlagung. Vor oder nach dem Eintauchen in das Salzbad kann eine zusätzliche Wärmebehandlung erfolgen. Bevorzugt erfolgt jedoch nur während des Eintauchens in das Salzbad eine Wärmebehandlung.

Bei einer Ausführung erfolgt die Wärmebehandlung im Salzbad in wenigstens zwei Schritten, wobei der zweite Schritt nach dem Einbringen von Funktionsmitteln, wie röntgensichtbaren Markierungen, und/oder nach Fügeprozessen erfolgt. Es hat sich gezeigt, dass bei einer Beschädigung der Mischoxidschicht durch mechanische oder thermische Fügeprozesse wie die Befestigung von röntgensichtbaren Materialien eine zweite Wärmehandlung diese Beschädigungen heilen kann.

Die Behandlungszeit, insbesondere des ersten Wärmebehandlungsschrittes beträgt vorzugsweise mindestens 1 min, vorzugsweise mindestens 2 min und/oder vorzugsweise höchstens 8 min, vorzugsweise höchstens 7 min, vorzugsweise höchstens 6 min, vorzugsweise höchstens 5 min, vorzugsweise höchstens 4 min, vorzugsweise höchstens 3 min. Die Dauer des zweiten Wärmebehandlungsschrittes kann mindestens 50% kürzer als die Dauer des ersten Wärmebehandlungsschrittes sein. Vorzugsweise beträgt die Dauer des ersten Wärmebehandlungsschrittes ca. 2min bis 4min und die Dauer des nachfolgenden zweiten Wärmebehandlungsschrittes ca. 20s bis 60s.

Die Temperaturobergrenze der Wärmebehandlung im Salzbad beträgt vorzugsweise 550°C, insbesondere 540°C, insbesondere 530°C, insbesondere 520°C. Die Untergrenze kann 400°C, insbesondere 420°C, insbesondere 440°C, insbesondere 460°C, insbesondere 480°C betragen.

Das Implantat ist vorzugsweise ein geflochtener Stent, kann jedoch auch ein anderes Implantat, beispielsweise ein Flow-Diverter oder ein Stent-Graft oder ein intravaskuläres Verschlussdevice oder ein intravaskuläres Coil sein.

Der Nickel-Anteil in der Mischoxidschicht beträgt vorzugsweise weniger als 6 Gew.-%, noch weiter vorzugsweise weniger als 3 Gew.-%, noch weiter vorzugsweise weniger als 2 Gew.-% jeweils wenigstens bis in eine Schichttiefe von 30% der Gesamtdicke der Mischoxidschicht, ausgehend von der Schichtoberfläche, insbesondere bis in eine Schichttiefe von 50% der Gesamtdicke der Mischoxidschicht, ausgehend von der Schichtoberfläche. Konkret kann sich der nickelarme Schichtbereich bis in eine Tiefe von 20nm - 40nm bei einer Schichtdicke von 60nm bis 100nm erstrecken. Dabei kann die Oberfläche der Mischoxidschicht überwiegend aus TiO₂ bestehen.

Ein Kontaktwinkel, d.h. der Benetzungswinkel der Oberfläche des Implantats beträgt bei Benetzung mit destilliertem Wasser vorzugsweise weniger als 90°, weiter bevorzugt weniger als 80°, noch weiter vorzugsweise weniger als 75°, und/oder mindestens 30%, vorzugsweise mindestens 60°. Bei einem derartigen Kontaktwinkel (Benetzungswinkel) ist die Biokompatibilität des Implantats vergleichsweise hoch. Weiterhin kann ein derartiger Kontaktwinkel besonders einfach durch die weiter oben beschriebene Reihenfolge der Herstellungsverfahrensschritte eingestellt werden. Erfindungsgemäß enthält das Salzbad ein Gemisch aus Kaliumnitrat und Natriumnitrit, wobei der Anteil an Kaliumnitrat größer als der Anteil an Natriumnitrit ist. Weitere Salze können (müssen aber nicht) enthalten sein. Es hat sich gezeigt, dass sich durch ein derartiges Salzbad eine Mischoxidschicht, enthaltend Titanoxinitrid und/oder Titannitrid, realisieren lässt, die sich durch günstige Oberflächeneigenschaften (geringe Rauheit) und eine hohe Biokompatibilität auszeichnet.

Es hat sich gezeigt, dass dichte und schützende Mischoxidschichten erzielt werden, was sich anhand des verbesserten Korrosionsverhaltens der Implantate zeigt, wenn der Anteil an Kaliumnitrat größer als der Anteil an Natriumnitrit ist. Konkret kann der das Salzbad folgende Bestandteile enthalten:
30-40 Gew.-% KNO₃
25-35 Gew.-% NaNO₂
Rest übliche Kohlenstoffverbindungen und Verunreinigungen,
wobei die Bedingung gilt, dass der Anteil an Kaliumnitrat größer als der Anteil an Natriumnitrit ist. Bei den Kohlenstoffverbindungen handelt es sich um Verbindungen, die dem Fachmann im Zusammenhang mit Salzbädern für die Wärmebehandlung bekannt sind. Vorzugsweise beträgt der Anteil an Kaliumnitrat 32-38 Gew.-%, insbesondere 34-36 Gew.-%. Der Anteil an Natriumnitrit kann 26-33 Gew.-%, insbesondere 27-30 Gew.-% .

Man nimmt an, dass der erhöhte Anteil an KNO₃, das eine höhere Zersetzungstemperatur als NaNO₂ aufweist, die Stickstoffanreicherung und Oxidierung begünstigt.

Die Mischoxidschicht kann mindestens 10 Gew.-%, weiter vorzugsweise mindestens 20 Gew.-%, weiter vorzugsweise mindestens 30 Gew.-%, weiter vorzugsweise mindestens 40 Gew.-%, weiter vorzugsweise mindestens 50 Gew.%. Titanoxinitrid und/oder Titanitrid aufweisen.

Bei dem Draht kann es sich um einen Endlosdraht handeln, der beispielsweise auf eine Spule und/oder von einer Spule gewickelt wird. Dadurch wird sowohl das Herstellungsverfahren - insbesondere des Elektropolierens - vereinfacht als auch ein einfach zu handhabendes Implantat realisiert.

Vorzugsweise wird aus dem mindestens einen Draht ein Geflecht gebildet. Besonders bevorzugt ist es dabei, wenn das Geflecht erst nach dem Elektropolieren bzw. allgemein vor der Wärmebehandlung gebildet wird. Bei dieser besonderen Ausführungsform wurde erkannt, dass im Stand der Technik üblicherweise die Kreuzungspunkte von (an sich bekannten) Drahtgeflechten eine Schwachstelle darstellen, da das Medium für die Elektropolitur in diesem Bereich der Überlappung der Drähte auf Grund von Abschattungseffekten nicht ausreichend wirken kann. Dadurch, dass erst nach dem Elektropolieren ein Geflecht gebildet wird, ist die Oberfläche des Drahtgeflechts insgesamt homogener und von vergleichsweise guter Biokompatibilität. Wenn der Draht vor dem Flechten elektropoliert wird, wird ein vergleichsweise gleichmäßiger Drahtdurchmesser erreicht, was die Mechanik des Funktionselementes, insbesondere Implantats und die Compliance positiv beeinflusst. Würde die Elektropolitur erst nach dem Flechten erfolgen, käme es im Bereich von Überkreuzungen der Drähte, in denen die Elektropolitur-Lösung nicht ausreichend gut in Kontakt mit den Drähten ist, zu einer Reduzierung des Abtrags beim Elektropolieren. Dies würde zu unregelmäßigen Drahtdurchmessern bzw ungleichen Ausbildung der Oberflächeneingenschaften führen.

Grundsätzlich können zur Herstellung des Geflechtes mehrere Drähte geflochten werden. Es kann beispielsweise auch ein einzelner Draht verwendet werden. Andere Drahtgebilde, bspw. gewickelte Drahtgebilde sind möglich.

Ferner wird im Rahmen der vorliegenden Anmeldung ein intravaskuläres Funktionselement offenbart, das in ein Gefäß einführbar ist und wenigstens einen Draht aus einer Legierung umfasst, die die Legierungselemente Nickel und Titan aufweist, vorzugsweise hergestellt nach einem Verfahren gemäß einem der vorhergehenden Ansprüche. Auf der Oberfläche des Drahtes ist eine Mischoxidschicht mit einer Schichtdicke von 15nm bis 100nm ausgebildet, die TiO₂ und wenigstens ein Nitrid, insbesondere Titanitrid und/oder Titanoxinitrid aufweist.

Vorzugsweise bildet der Draht ein Drahtgebilde, insbesondere ein Coil zur Aneurysmabehandlung oder ein Geflecht, bei dem sich wenigstens ein Abschnitt, insbesondere mehrere Abschnitte, des verformten Drahtes oder mehrere Drähte überkreuzen und/oder berühren.

Weiter vorzugsweise ist die Mischoxidschicht im Bereich der Drahtüberkreuzung und/oder auf den Kontaktflächen der sich berührenden Drahtabschnitte oder Drähte ausgebildet.

In einem weiteren Ausführungsbeispiel beträgt die Schichtdicke mindestens 30nm, insbesondere mindestens 35nm, insbesondere mindestens 40nm, insbesondere mindestens 45nm, insbesondere mindestens 50nm, insbesondere mindestens 55nm.

Vorzugsweise beträgt die Schichtdicke höchstens 95nm, insbesondere höchstens 85nm, insbesondere höchstens 80nm, insbesondere höchstens 75nm, insbesondere höchstens 70nm, insbesondere höchstens 65nm, insbesondere höchstens 60nm.

In einem weiteren Ausführungsbeispiel bildet der Peak der Sauerstoffkonzentration in der Mischoxidschicht ein Plateau.

In einem weiteren Ausführungsbeispiel beträgt das Verhältnis der Intensitäten zwischen Stickstoff und Sauerstoff N/O im Bereich des Sauerstoffplateaus höchstens 1:2,0, insbesondere aber von 1:2,5 bis 1:10 und nimmt zum Drahtmetallkörper hin ab, wobei die Intensität jeweils durch Augerelektronenspektroskopie (AES) ermittelt wird.

Vorzugsweise liegt Stickstoff ausgehend von der Schichtoberfläche bis in eine Tiefe der Mischoxidschicht von bis zu 2/6 der Gesamtdicke der Mischoxidschicht, insbesondere bis zu 3/6, insbesondere bis zu 4/6 der Gesamtdicke der Mischoxidschicht vor.

Vorteilhafterweise beträgt ein Ni-Anteil der Mischoxidschicht in einem Bereich ausgehend von der Schichtoberfläche bis in eine Schichttiefe von 30% der Gesamtdicke der Mischoxidschicht höchstens 6 Gew.-%. Vorzugsweise hat die Oberflächenschicht einen Nickel-Anteil von weniger als 3 Gew.-%, noch weiter vorzugsweise von weniger als 2 Gew.-%.

Weiter vorzugsweise ist im inneren Bereich der Mischoxidschicht, der an den Drahtmetallkörper angrenzt, eine Anreicherung von Nickeloxid ausgebildet.

Zu den Vorteilen des Funktionselements wird auf die im Zusammenhang mit dem Herstellungsverfahren erläuterten Vorteile verwiesen.

Insgesamt wird durch die oben beschriebenen Maßnahmen insbesondere die Nickel-Freisetzung von Implantaten minimiert, die Radialkraft von medizinischen Implantaten (z.B. aus Nitinol) optimiert, das Werkstoff-Ermüdungsverhalten des Implantates (z.B. aus Nitinol) verbessert, das Korrosionsverhalten des Implantates (z.B. aus Nitinol) verbessert, die Oberflächenrauheit des Implantats (z.B. aus Nitinol) verringert sowie die Thrombogenität von medizinischen Implantaten (z.B. aus Nitinol) verbessert. Außerdem kommt es (aufgrund einer geringen bzw. nicht vorhandenen Porosität) zu einer äußerst geringen Ablagerung von Proteinen und weiteren Blutbestandteilen, die zur Bildung von Thromben führen kann. An Stelle von Geflechten können allgemein Drahtgebilde zum Einsatz kommen, die mindestens eine Kontaktstelle zwischen zwei Drähten haben (Vorrichtungen z.B., die aus Wire-Forming hergestellt werden, oder anderen Textilverfahren).

Sollte ein Parameter (z.B. die Schichtdicke) variieren, kann der jeweilige Maximalwert oder der (geometrische) Mitteilwert gemeint sein.

Die Erfindung wird im Folgenden anhand von Ausführungsbeispielen unter Bezugnahme auf die beigefügten, schematischen Zeichnungen näher erläutert. Darin zeigen:
- Fig. 1: mehrere Drähte in einem Elektropolitur-Bad;
- Fig. 2: ein schematisches Geflecht aus mehreren Drähten;
- Fig. 3: das Geflecht gemäß Fig. 2 in einem Salzbad;
- Fig. 4: ein Tiefenprofil einer Mischoxidschicht eines Funktionselementes nach einem erfindungsgemäßen Ausführungsbeispiel mit einer Schichtdicke von ca. 60nm (Probe1.6pro);
- Fig. 5: ein Tiefenprofil einer Mischoxidschicht eines Funktionselementes nach einem Vergleichsbeispiel mit einer Schichtdicke von ca. 220nm (Auger2.5+3.pro);
- Fig. 6: eine Korrosionskurve (0611-170-01) eines Funktionselementes als Vergleichsbeispiel, bei dem ein nicht-elektropolierter Draht verwendet wird;
- Fig. 7: eine Korrosionskurve (1586-170-04) eines Funktionselementes nach einem erfindungsgemäßen Ausführungsbeispiel;
- Fig. 8: eine REM-Aufnahme einer unbehandelten Vergleichsprobe und
- Fig. 9: eine REM-Aufnahme einer Drahtoberfläche, die nach einer Ausführung des erfindungsgemäßen Verfahrens erzeugt ist.

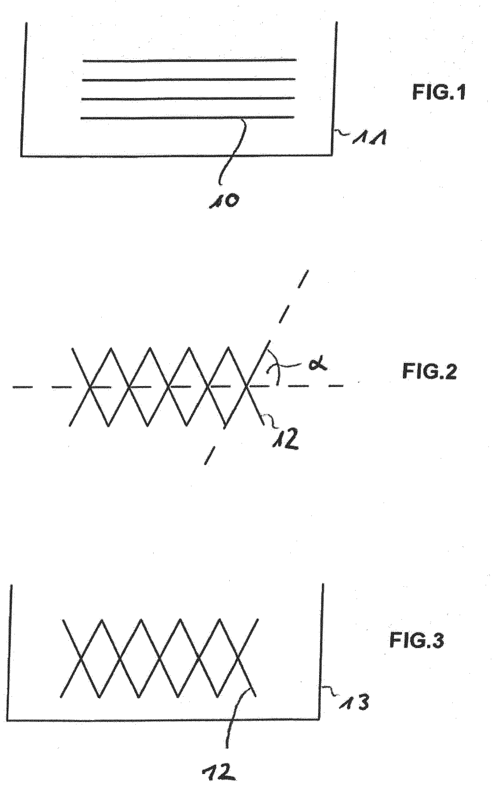

Fig. 1 zeigt einen ersten Schritt für die Herstellung eines Stents. Mehrere Drähte 10 (in der schematischen Zeichnung vier Drähte) werden in ein Elektropolitur-Bad 11 eines Elektrolyten eingetaucht. Dieser Schritt kann wie in der US 2004/0117001 A1, abgesehen von der zeitlichen Reihenfolge, durchgeführt werden.

Fig. 2 zeigt (schematisch) ein Geflecht 12 aus den Drähten 10. Das Geflecht 12 ist dabei im aufgeklappten Zustand dargestellt, so dass die gesamte Umfangsfläche des Geflechts 12 in der Zeichnungsebene abgebildet ist. Nach dem Schritt des Flechtens wird das Geflecht 12 in ein Salzbad 13 eingetaucht und wärmebehandelt (sh. Fig. 3). In dem Salzbad 13 erhält das Geflecht 12 bzw. der Stent seine endgültige Struktur einschließlich der passivierten Oberfläche. Dies schließt nicht aus, dass noch weitere Bearbeitungsschritte nachfolgen können.

### Beispiel

Die Erfindung wird beispielhaft anhand eines Funktionselements erläutert, das aus einer binären NiTi-Legierung, wie Nitinol, hergestellt ist. Andere NiTi enthaltende Legierung sind möglich. Dabei wird die Modifikation der Oberfläche durch die thermische Behandlung im Salzbad dargestellt, die für die Einstellung der Stickstoffkonzentration in der TiO₂ Mischoxidschicht verantwortlich ist. Das Grundelement des Funktionselementes, nämlich der Draht, wird im ersten Schritt elektropoliert. Die Elektropolitur kann wie im Stand der Technik üblich durchgeführt werden, bspw. bei einer Temperatur T < 20° unter Verwendung einer Methanol-Schwefelsäure-Lösung. Auf dem elektropolierten Draht bildet sich durch Kontakt mit der Umgebungsluft spontan eine homogene natürliche Oxidschicht mit einer Schichtdicke von ca. 5nm.

Im zweiten Schritt wird aus dem elektropolierten Draht das Funktionselement, ein Stent, geflochten.

Im dritten Schritt wird das Funktionselement zur Erhöhung der Schichtdicke im Salzbad wärmebehandelt. Dabei wird eine Salzbadzusammensetzung verwendet, die aus folgenden Komponenten besteht:
ca. 35-36 Gew.-% KNO₃
ca. 27-29 Gew.-% NaNO₂
Rest übliche Kohlenstoffverbindungen und Verunreinigungen.

Es hat sich gezeigt, dass gute Ergebnisse erzielt werden können, wenn der Anteil an Kaliumnitrat größer als der Anteil an Natriumnitrit im Salzbad ist.

Die Prozesstemperaturen betragen ca. 490° C - 510° C . Im ersten Behandlungsschritt wird das Funktionselement ca. 2 bis 3min in das Salzbad getaucht. Dabei erfolgt die Ausbildung der Oxidschicht. Die Behandlungsdauer im zweiten Schritt beträgt ca. 30sec. oder weniger.

### Messverfahren

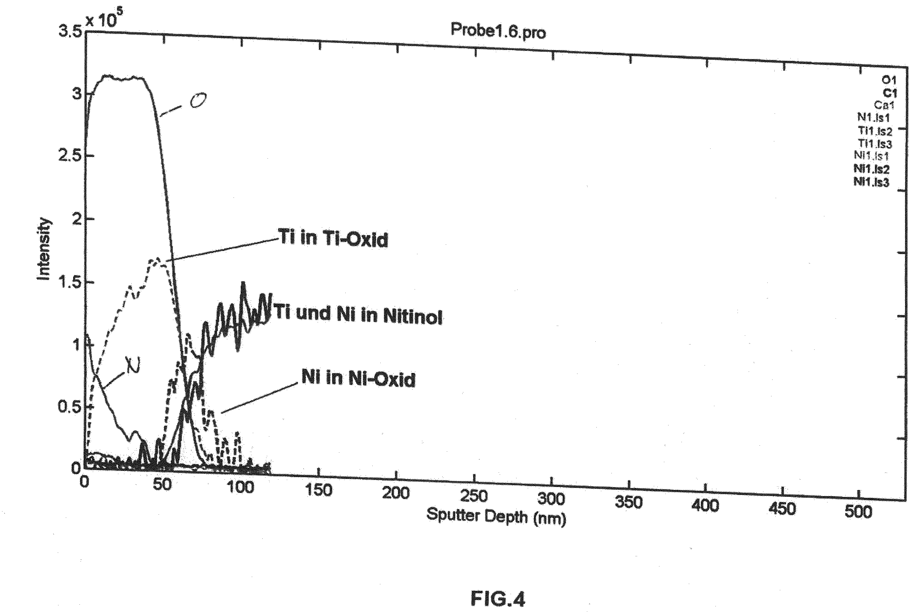

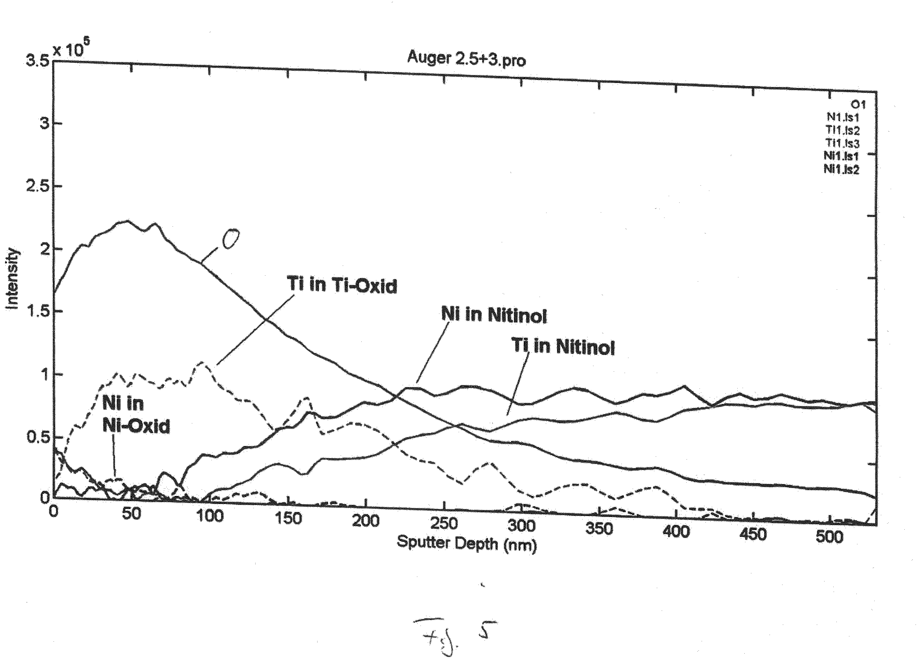

Die Messungen zur Erstellung des AES-Tiefenprofils gemäß Fig. 4, 5 wurden mit folgenden Parametern durchgeführt:
Primärelektronenenergie (Anregung): 5keV
Strahlstrom: 20nA
Elektronenstrahlraster (analysierter Bereich): 20µm x 20µm
Ionenstrahlenergie: 3keV (Fig. 4, Probe 1.6.pro, jeweils behandelt)
Strahlstrom: 2µA
Abtragrate: 59,3nm/min.
Ionenstrahlraster: 0,8mm x 0,8mm
Ionenstrahlenergie: 1keV (Fig. 5, Auger2.5+3.pro, unbehandelt)
Strahlstrom: 0,5µA
Abtragrate: 8,24nm/min.
Ionenstrahlraster: 0,8mm x 0,8mm
Probenwinkel (Elektronenstrahl - Probennormale): 30°

Für die Bestimmung der Intensitäten wurden folgende Elementpeaks verwendet:
Ti1: Ti LMM bei 390 eV
Ti2: Ti LMM bei 421 eV
N1: N KLL bei 389 eV
Ni1: Ni LMM bei 849 eV
O1: KLL bei 510 eV

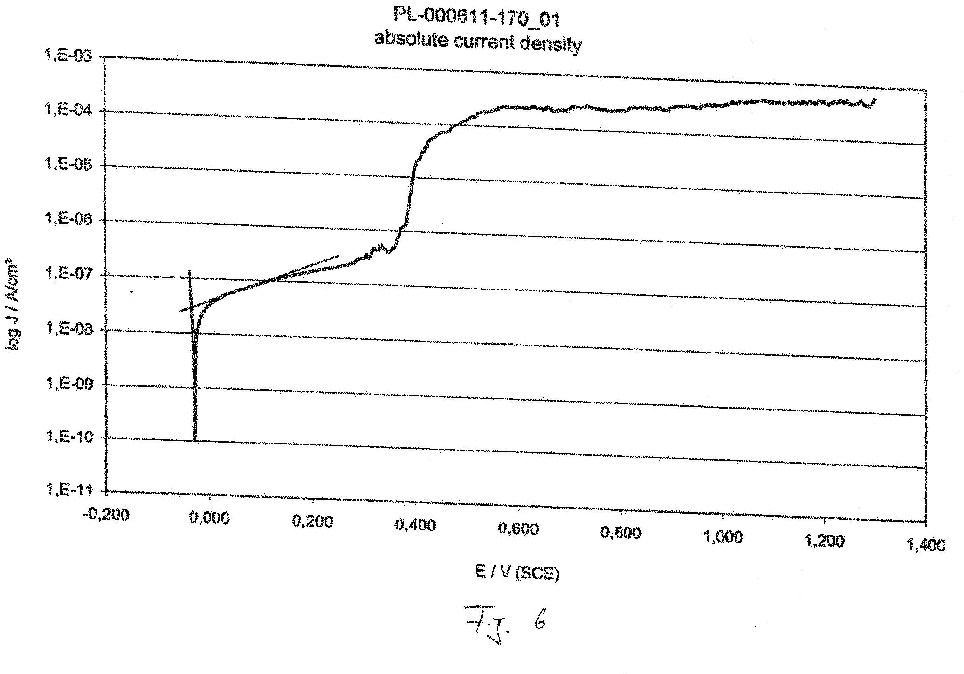

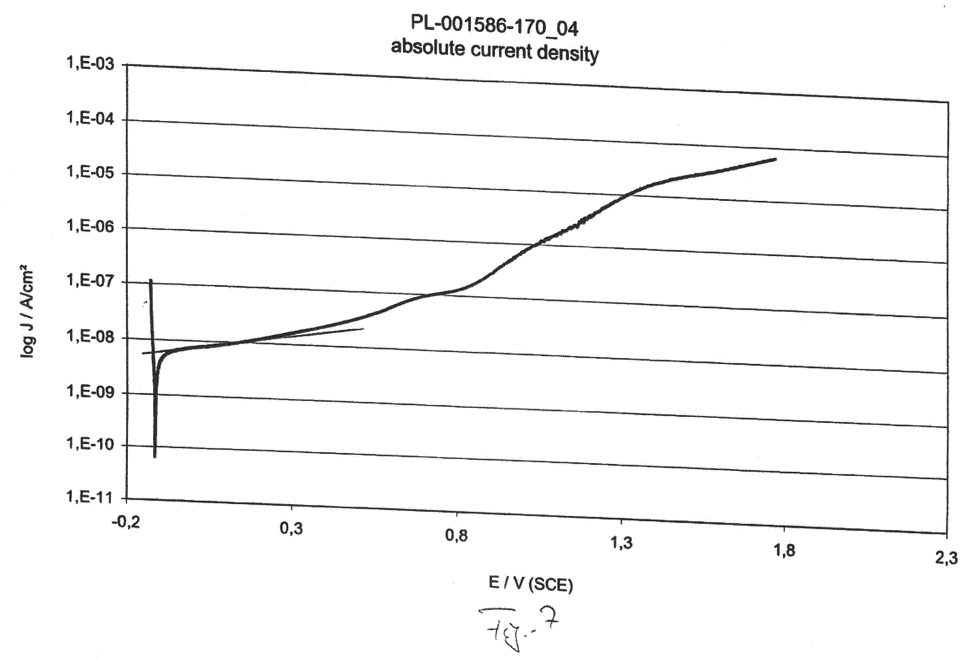

Die Messungen für die Korrosionskurven gemäß Fig. 6, 7 wurden nach ASTM F2129 "Standard Test Method for Conducting Cyclic Potentiodynamic Polarization Measurements to Determine the Corrosion Susceptibility of Small Implant Devices" durchgeführt.

### Ergebnisse und Diskussion

Das Tiefenprofil gemäß Fig. 4, bei der die Sputtertiefe auf 500 nm normiert ist, zeigt den Konzentrationsverlauf der Schichtelemente, der sich nach der Durchführung des vorstehend erläuterten Verfahrens ergibt. Dabei wird generell die Schichtdicke aus den Sputterparametern bestimmt. Alternativ zur Bestimmung der Schichtdicke mit 50% vom "Peak-Wert" TiO₂ gerechnet werden. Demnach hat die Schicht eine Dicke von ca. 60nm, was sich aus der Schnittstelle des SauerstoffPeaks und der Peaks für metallisches Ti und Ni ergibt. Markiert sind in Fig. 4 die folgenden Peaks:
Sauerstoff
Stickstoff
Ti im Titanoxid
Titan (metallisches Titan)
Ni im Ni-Oxid
Ni

Besonders prägnant ist die Ausbildung des Sauerstoffpeaks als ein Plateau. Das Plateau befindet sich in einer Schichttiefe zwischen etwa 10nm und 40nm. Eine mögliche Erklärung wird darin gesehen, dass der Sauerstoff zum äusseren Teil der Schicht hin sich auch mit Stickstoff verbindet und zum inneren Teil der Schicht hin sich dann mit Titan verbindet. Der Stickstoff ist als chemische Verbindung in die Schicht eingebaut, und zwar als Titanoxinitrid. Das ergibt sich aus dem Verlauf des Sauerstoffsignals, das ein Plateau bildet. Ggf. ist der Stickstoff zusätzlich auch noch als Titanitrid vorhanden. Allgemein bedeutet der Plateauverlauf, dass die Sauerstoffintensität bereichsweise, insbesondere über eine Schichttiefe von mindestens 10nm konstant ist.

Das Verhältnis der Intensitäten zwischen Stickstoff und Sauerstoff N/O in der Schicht beträgt im Bereich des Sauerstoffplateaus in der größten Ausprägung ca. 1:3; 1:6; 1:10, wobei das Verhältnis 1:3 in der unmittelbaren Randschicht der Schichtaußenfläche (ca. 5nm-10nm), das Verhältnis 1:6 in etwa bei einer Schichttiefe von ca. 20-25nm und das Verhältnis 1:10 in etwa bei einer Schichttiefe von ca. 35-40 nm vorliegt. An der Schichtoberfläche beträgt das Verhältnis ca. 1:2,5.

Aus Fig. 4 ergibt sich ferner, dass es im inneren Teil der Schicht, also nah am metallischen Drahtkörper, eine deutliche Anreicherung von Ni-Oxid gibt. Der Rest der Schicht weist kaum Nickel auf. Insbesondere die äußere Randschicht ist nickelarm. Dieser Konzentrationsverlauf könnte durch den Stickstoff bedingt sein, der sich ggf. bevorzugt gegenüber Ni mit dem Sauerstoff verbindet und somit eine Nickel-Anreicherung im äußeren Teil der Schicht unterbindet.

Fig. 5 zeigt das Tiefenprofil einer unbehandelten Probe mit einer Oxidschichtdicke von ca. 220nm (s. Schnittpunkt Ni/O). Die untersuchte Oxidschicht wurde durch die Wärmebehandlung bei der Drahtherstellung gebildet. Bei der Probe gemäß Fig. 5 wurde keine Vorbehandlung durchgeführt, d.h. die herstellungsbedingte Oxidschicht wurde auf der Oberfläche des Drahtes belassen. Fig. 5 zeigt, dass der Sauerstoffverlauf kein Plateau bildet. Die Sauerstoffintensität nimmt bis ca. 50nm zu und fällt dann ab. Im Unterschied zur Schicht gemäß Fig. 4 ist außerdem eine leichte Nickel-Oxid-Anreicherung im oberflächennahen Bereich der Schicht festzustellen. Die Nickel-Oxidanreicherung im metallkörpernahen Bereich der Schicht fehlt. Die Stickstoffintensität in der Schicht ist insgesamt deutlich kleiner als bei der Schicht gemäß Fig. 4.

Die Bewertung des Schutzverhaltens der Schicht erfolgt anhand der Korrosionskurven gemäß Fig. 6, 7, aus denen das elektrochemische Verhalten der Schichten und damit die interessierenden Schichteigenschaften, wie zB das Freisetzen von Nickel-Ionen, abgeleitet werden können. In den Fig. 6, 7 ist die Stromdichte J / A/cm² über der Spannung E/V (SCE) aufgetragen.

Fig. 7 zeigt die Korrosionskurve (1586-170-04) einer erfindungsgemäß hergestellten Schicht, die eine sehr niedrige Korrosionsstromdichte (<1x10-8 A/cm2) aufweist. Dies bedeutet, dass die Schicht eine geringe Durchlässigkeit für Metallionen und damit gute Schutzwirkung zeigt. Besonders wichtig ist, wie sich aus dem nahezu linearen Anstieg ergibt, dass kein Durchbruch, dh keine Pitting Korrosion auftritt. Die Schichteigenschaften sind demnach hervorragend.

Im Unterschied dazu liegt gemäß Fig. 6 der Korrosionsstrom bei der herkömmlich hergestellten Schicht über 1x10-7 A/cm². Bei etwa 400mV sind Durchbrüche zu beobachten, die auf den Beginn von Pitting-Korrosion, also Lochfraß hindeuten.

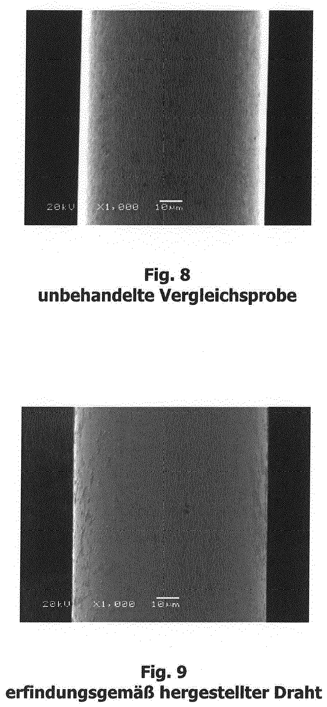

Die guten Oberflächeneigenschaften ergeben sich aus dem Vergleich zwischen der in Fig. 8 dargestellten Oberfläche eines unbehandelten Drahtes mit einer herstellungsbedingten Oxidschicht nd der in Fig. 9 gezeigten Oberfläche eines nach der Erfindung wärmebehandelten Drahtes. Die Oxidschicht des Drahtes gemäß Fig. 9 ist gleichmäßig dicht und porenfrei.

Mit dem erfindungsgemäßen Verfahren lassen sich sehr korrosionsstabile und feste Mischoxidschichten herstellen, die eine gute Schutzwirkung entfalten und sicher vor Abrieb schützen.

### Bezugszeichenliste

- α: Flechtwinkel
- 10: Draht
- 11: Elektropolitur-Bad
- 12: Geflecht
- 13: Salzbad

## Patentansprüche

1. Verfahren zur Herstellung eines intravaskulären Funktionselementes, das in ein Hohlorgan einführbar ist und wenigstens einen Draht (10) aus einer Legierung umfasst, die die Legierungselemente Nickel und Titan aufweist, mit den folgenden Schritten:
- Bereitstellen eines Metallkörpers des Drahtes (10) mit metallischer Oberfläche, dann
- Ausbilden einer ersten Oxidschicht auf der metallischen Oberfläche des Metallkörpers, dann
- Durchführen einer Wärmebehandlung des Drahtes (10) in einem Stickstoff enthaltenden Salzbad zur thermischen Ausbildung einer zweiten Mischoxidschicht auf der ersten Oxidschicht, wobei die Schritte zum Ausbilden der ersten und zweiten Oxidschicht derart ausgeführt werden, dass die Gesamtschichtdicke von 15nm bis 100nm beträgt und die Mischoxidschicht TiO₂ und wenigstens Titanoxinitrid und/oder Titanitrid aufweist, wobei das Salzbad ein Gemisch aus Kaliumnitrat und Natriumnitrit, enthält, wobei der Anteil an Kaliumnitrat größer als der Anteil an Natriumnitrit ist.

2. Verfahren nach Anspruch 1,
**dadurch gekennzeichnet, dass**
eine Gesamtschichtdicke von mindestens 30nm, insbesondere mindestens 35nm, insbesondere mindestens 40nm, insbesondere mindestens 45nm, insbesondere mindestens 50nm, insbesondere mindestens 55nm, ausgebildet wird.

3. Verfahren nach Anspruch 1 oder 2,
**dadurch gekennzeichnet, dass**
eine Gesamtschichtdicke von höchstens 95nm, insbesondere höchstens 85nm, insbesondere höchstens 80nm, insbesondere höchstens 75nm, insbesondere höchstens 70nm, insbesondere höchstens 65nm, insbesondere höchstens 60nm, ausgebildet wird.

4. Verfahren nach einem der vorhergehenden Ansprüche,
**dadurch gekennzeichnet, dass**
der Draht zur Herstellung eines Drahtgebildes vor der Wärmebehandlung zur Ausbildung der Mischoxidschicht verformt wird, wobei sich wenigstens ein Abschnitt, insbesondere mehrere Abschnitte des verformten Drahtes überkreuzen und/oder berühren.

5. Verfahren nach Anspruch 4,
**dadurch gekennzeichnet, dass**
der wenigstens eine Draht oder mehrere Drähte zu einem Geflecht (12) verflochten werden.

6. Verfahren nach Anspruch 4,
**dadurch gekennzeichnet, dass**
der Draht zu einem Coil gewunden wird.

7. Verfahren nach einem der vorhergehenden Ansprüche ,
**dadurch gekennzeichnet, dass**
das Salzbad folgende Bestandteile enthält
30-40 Gew.-% KNO₃
25-35 Gew.-% NaNO₂
Rest übliche Kohlenstoffverbindungen und Verunreinigungen,
wobei die Bedingung gilt, dass der Anteil an Kaliumnitrat größer als der Anteil an Natriumnitrit ist.

8. Verfahren nach einem der vorhergehenden Ansprüche,
**dadurch gekennzeichnet, dass**
die Wärmebehandlung im Salzbad in wenigstens zwei Schritten erfolgt, wobei der zweite Schritt nach dem Einbringen von Funktionsmitteln, wie röntgensichtbaren Markierungen, und/oder nach Fügeprozessen erfolgt.

9. Verfahren nach einem der vorhergehenden Ansprüche, wobei die Temperaturobergrenze der Wärmebehandlung im Salzbad 550°C, insbesondere 540°C, insbesondere 530°C, insbesondere 520°C, und die Untergrenze 400°C, insbesondere 420°C, insbesondere 440°C, insbesondere 460°C, insbesondere 480°C, beträgt.

10. Verfahren nach einem der vorhergehenden Ansprüche,
**dadurch gekennzeichnet, dass**
zum Bereitstellen des Metallkörpers des Drahtes (10) mit metallischer Oberfläche der Draht elektropoliert wird.

11. Verwendung eines Salzbades zur Wärmebehandlung eines intravaskulären Funktionselementes, das in ein Hohlorgan einführbar ist, wobei das Salzbad folgende Bestandteile enthält
30-40 Gew.-% KNO₃
25-35 Gew.-% NaNO₂
Rest übliche Kohlenstoffverbindungen und Verunreinigungen,
wobei die Bedingung gilt, dass der Anteil an Kaliumnitrat größer als der Anteil an Natriumnitrit ist.

## Claims

1. A method for manufacturing an intravascular function element which can be inserted into a hollow organ and comprises at least one wire (10) made of an alloy containing the alloy elements nickel and titanium, comprising the following steps:
- providing a metal body of the wire (10) with a metallic surface, then
- forming a first oxide layer on the metallic surface of the metal body, then
- performing a heat treatment of the wire (10) in a salt bath containing nitrogen for thermal development of a second mixed oxide layer on the first oxide layer, wherein the steps for forming the first and second oxide layers are carried out in such a way that the total layer thickness amounts to 15 nm to 100 nm, and the mixed oxide layer contains TiO₂ and at least titanium oxynitride and/or titanium nitride, wherein the salt bath contains a mixture of potassium nitrate and sodium nitrite, wherein the amount of potassium nitrate is greater than the amount of sodium nitrite.

2. The method according to claim 1,
**characterized in that**
a total layer thickness of at least 30 nm, in particular at least 35 nm, in particular at least 40 nm, in particular at least 45 nm, in particular at least 50 nm, in particular at least 55 nm is formed.

3. The method according to claim 1 or 2,
**characterized in that**
a total layer thickness of at most 95 nm, in particular at most 85 nm, in particular at most 80 nm, in particular at most 75 nm, in particular at most 70 nm, in particular at most 65 nm, in particular at most 60 nm is formed.

4. The method according to any one of the preceding claims,
**characterized in that**
the wire for producing a wire structure is deformed before the heat treatment in order to form the mixed oxide layer, wherein at least one section, in particular several sections of the deformed wire intersect and/or come in contact.

5. The method according to claim 4,
**characterized in that**
at least one wire is or several wires are braided to form a braid (12).

6. The method according to claim 4,
**characterized in that**
the wire is wound up to form a coil.

7. The method according to any one of the preceding claims,
**characterized in that**
the salt bath contains the following ingredients:
30 - 40 % by weight KNO₃,
25 - 35 % by weight NaNO₂,
the remainder consisting of the usual carbon compounds and impurities,
wherein the condition holds that the amount of potassium nitrate is greater than the amount of sodium nitrite.

8. The method according to any one of the preceding claims,
**characterized in that**
the heat treatment in the salt bath takes place in at least two steps,
wherein the second step takes place after introducing function means, such as radiopaque markings, and/or after joining operations.

9. The method according to any one of the preceding claims, wherein the upper temperature limit in the heat treatment in the salt bath is 550 °C, in particular 540 °C, in particular 530 °C, in particular 520 °C, and the lower limit is 400 °C, in particular 420 °C, in particular 440 °C, in particular 460 °C, in particular 480 °C.

10. The method according to any one of the preceding claims,
**characterized in that**
the wire is electropolished to provide the metal body of the wire (10) with a metallic surface.

11. A use of a salt bath for a thermal treatment of an intravascular function element, which can be inserted into a hollow organ, wherein the salt bath contains the following ingredients:
30 - 40 % by weight KNO₃,
25 - 35 % by weight NaNO₂,
the remainder comprising the usual carbon compounds and impurities,
wherein there is the applicable condition that the amount of potassium nitrate is greater than the amount of sodium nitrite.

## Revendications

1. Procédé pour la fabrication d'un élément fonctionnel intravasculaire, lequel peut être inséré dans un organe creux et comprend au moins un fil (10) en un alliage qui présente les éléments d'alliage de nickel et de titane, avec les étapes suivantes :
- mise à disposition d'un corps métallique du fil (10) avec une surface métallique, puis
- formation d'une première couche d'oxyde sur la surface métallique du corps métallique, puis
- réalisation d'un traitement thermique du fil (10) dans un bain de sel contenant de l'azote pour la formation thermique d'une deuxième couche d'oxyde mixte sur la première couche d'oxyde, dans lequel les étapes de formation de la première et la deuxième couche d'oxyde sont réalisées de manière à ce que l'épaisseur de couche totale comporte de 15 nm à 100 nm et présente la couche d'oxyde mixte TiO₂ et au moins de l'oxynitrure de titane et/ou du nitrure de titane, dans lequel le bain de sel contient un mélange de nitrate de potassium et de nitrite de sodium, dans lequel la part de nitrate de potassium est supérieure à la part de nitrite de sodium.

2. Procédé selon la revendication 1,
**caractérisé en ce**
**qu'**une épaisseur de couche totale d'au moins 30 nm, en particulier d'au moins 35 nm, en particulier d'au moins 40 nm, en particulier d'au moins 45 nm, en particulier d'au moins 50 nm, en particulier d'au moins 55 nm est réalisée.

3. Procédé selon la revendication 1 ou 2,
**caractérisé en ce**
**qu'**une épaisseur de couche totale d'un maximum de 95 nm, en particulier d'un maximum de 85 nm, en particulier d'un maximum de 80 nm, en particulier d'un maximum de 75 nm, en particulier d'un maximum de 70 nm, en particulier d'un maximum de 65 nm, en particulier d'un maximum de 60 nm est réalisée.

4. Procédé selon l'une des revendications précédentes, **caractérisé en ce que**
le fil pour la fabrication d'une structure à fil est déformé avant le traitement thermique pour la formation de la couche d'oxyde mixte, dans lequel au moins un tronçon, en particulier plusieurs tronçons du fil déformé se croisent et/ou se touchent.

5. Procédé selon la revendication 4,
**caractérisé en ce que**
l'au moins un fil ou plusieurs fils sont entrelacés pour donner un entrelacement (12).

6. Procédé selon la revendication 4,
**caractérisé en ce que**
le fil est enroulé pour donner une bobine.

7. Procédé selon l'une des revendications précédentes, **caractérisé en ce que**
le bain de sel contient les constituants suivants :
30 - 40 % en poids KNO₃
25 - 35 % en poids NaNO₂
le reste étant des composés de carbone usuels et des impuretés,
dans lequel il y a pour condition que la part de nitrate de potassium est supérieure à la part de nitrite de sodium.

8. Procédé selon l'une des revendications précédentes, **caractérisé en ce que**
le traitement thermique dans le bain de sel s'effectue en au moins deux étapes,
dans lequel la deuxième étape s'effectue après l'introduction de moyens fonctionnels, tels des marquages visibles à la radiographie, et/ou après des processus d'assemblage.

9. Procédé selon l'une des revendications précédentes, dans lequel la limite supérieure de température du traitement thermique dans le bain de sel comporte 550 °C, en particulier 540 °C, en particulier 530 °C, en particulier 520 °C, et la limite inférieure comporte 400 °C, en particulier 420 °C, en particulier 440 °C, en particulier 460 °C, en particulier 480 °C.

10. Procédé selon l'une des revendications précédentes, **caractérisé en ce que**,
pour la mise à disposition du corps métallique du fil (10) avec surface métallique, le fil est soumis à un électropolissage.

11. Utilisation d'un bain de sel pour le traitement thermique d'un élément fonctionnel intravasculaire, lequel peut être inséré dans un organe creux, dans laquelle le bain de sel contient les constituants suivants
30 - 40 % en poids KNO₃
25 - 35 % en poids NaNO₂
le reste étant des composés de carbone usuels et des impuretés,
dans lequel il y a pour condition que la part de nitrate de potassium est supérieure à la part de nitrite de sodium.
